# EUROPEAN PATENT APPLICATION

(11) **EP 2 149 549 A1**
(43) Date of publication of application: **03.02.2010**
(21) Application number: 08764487.8
(22) Date of filing: 22.05.2008
(51) Int. Cl.: C07D 207/335, A61K 31/381, A61K 31/40, A61P 1/16, A61P 37/06, C07D 333/22

(54) **PHARMACEUTICAL COMPOSITION FOR PREVENTION OR TREATMENT OF HEPATITIS**

(30) Priority: 25.05.2007 JP 2007138771
(71) Applicant: Daiichi Sankyo Company, Limited, Chuo-ku Tokyo 103-8426 (JP)
(72) Inventor: SHIMOZATO, Takaichi, Tokyo 134-8630 (JP); KAGARI, Takashi, Tokyo 134-8630 (JP); DOI, Hiromi, Tokyo 134-8630 (JP)
(74) Representative: Fairbairn, Angus Chisholm
(86) International application number: PCT/JP2008/059414
(87) International publication number: WO 2008/146691

(57) **Abstract**

The present invention provides a pharmaceutical composition for prophylaxis or treatment of hepatitis. Solution

A pharmaceutical composition for prophylaxis or treatment of hepatitis, comprising a compound represented by the general formula (I): 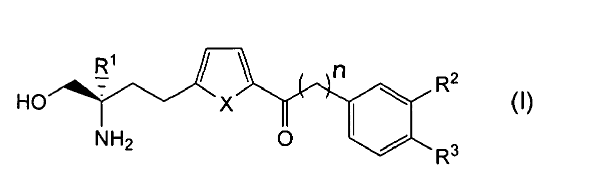 wherein
R¹ represents a C₁-C₄ alkyl group,
R² and R³ which are the same or different represent a hydrogen atom, a C₁-C₄ alkyl group, or a C₁-C₄ alkoxy group,
X represents a sulfur atom or a nitrogen atom substituted by a methyl group, and
n represents an integer of 3 or 4,
or a pharmacologically acceptable salt thereof as an active ingredient.

## Description

### Technical Field

The present invention relates to a pharmaceutical composition for prophylaxis or treatment of hepatitis, comprising an amino alcohol compound as an active ingredient.

### Background Art

The present applicant discloses compounds, as shown below, as pharmaceutical compositions excellent as prophylactic or therapeutic drugs for diseases associated with immunological effects, such as rejection of organ or skin transplants, rheumatoid arthritis, psoriasis, inflammatory bowel disease, multiple sclerosis, and other autoimmune diseases.

Specifically, these compounds are:
(1) a compound represented by the general formula (a) : 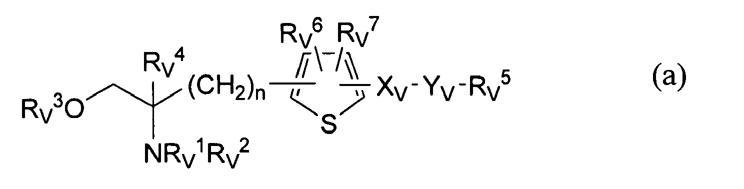 wherein Rᵥ¹ and Rᵥ² represent a hydrogen atom, a protecting group for an amino group, or the like; Rᵥ³ represents a hydrogen atom, a protecting group for a hydroxy group, or the like; Rᵥ⁴ represents a lower alkyl group; n represents an integer of 1 to 6; Xᵥ represents an ethylene group or the like; Yᵥ represents a C₁-C₁₀ alkylene group or the like; Rᵥ⁵ represents an aryl group, a substituted aryl group, or the like; and Rᵥ⁶ and Rᵥ⁷ represent a hydrogen atom or the like, provided that when Rᵥ⁵ is a hydrogen atom, Yᵥ represents a group other than a single bond and a linear C₁-C₁₀ alkylene group (see Patent Document 1); and
(2) a compound represented by the general formula (b) : 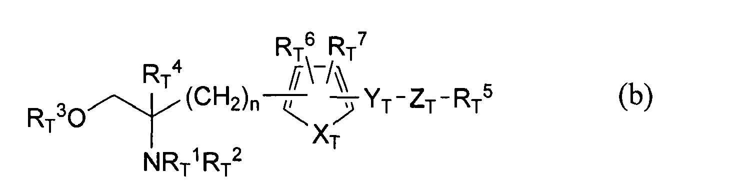
wherein R_{T}¹ and R_{T}² represent a hydrogen atom, a protecting group for an amino group, or the like; R_{T}³ represents a hydrogen atom, a protecting group for a hydroxy group, or the like; R_{T}⁴ represents a lower alkyl group; n represents an integer of 1 to 6; X_{T} represents an oxygen atom or a nitrogen atom which is not substituted or a nitrogen atom which is substituted by lower alkyl group or the like; Y_{T} represents an ethylene group or the like; Z_{T} represents an alkylene group having 1 to 10 carbon atoms, or the like; R_{T}⁵ represents an aryl group, a substituted aryl group, or the like; and R_{T}⁶ and R_{T}⁷ represent a hydrogen atom or the like, provided that when R_{T}⁵ is a hydrogen atom, Z_{T} represents a group other than a single bond and a linear alkylene group having 1 to 10 carbon atoms (see Patent Document 2).
[Patent Document 1] International Publication No. WO 2002/06228
[Patent Document 2] International Publication No. WO 2003/059880

### Disclosure of the Invention

### Problems to be Solved by the Invention

An object of the present invention is to provide a pharmaceutical composition useful for prophylaxis or treatment of hepatitis.

### Means for Solving the Problems

The present inventors have conducted diligent studies for years to attain the object and consequently found that, among amino alcohol compounds, a compound having a particular structure has high efficacy for prophylaxis or treatment of hepatitis. The present invention has been completed based on these findings.

Specifically, the present invention provides a pharmaceutical composition for prophylaxis or treatment of hepatitis, comprising a particular amino alcohol compound as an active ingredient. A preferred aspect of this invention provides the pharmaceutical composition, wherein the hepatitis is T cell-dependent hepatitis (viral hepatitis or autoimmune hepatitis), alcoholic hepatitis, or drug-induced hepatitis (more preferably, the hepatitis is T cell-dependent hepatitis (viral hepatitis or autoimmune hepatitis)).

Specifically, the present invention provides:
(1) a pharmaceutical composition for prophylaxis or treatment of hepatitis, comprising a compound represented by the general formula (I): 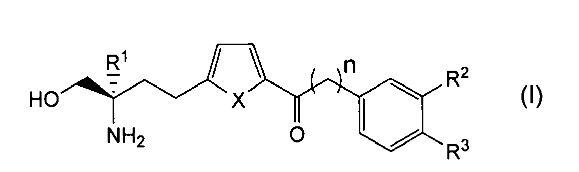 wherein
   R¹ represents a C₁-C₄ alkyl group,
   R² and R³ which are the same or different represent a hydrogen atom, a C₁-C₄ alkyl group, or a C₁-C₄ alkoxy group,
   X represents a sulfur atom or a nitrogen atom substituted by a methyl group, and
   n represents an integer of 3 or 4,
   or a pharmacologically acceptable salt thereof, as an active ingredient.
   Preferably, the present invention provides:
(2) the pharmaceutical composition according to (1), wherein R¹ is a methyl group, and R² and R³ which are the same or different are a hydrogen atom, a methyl group, or a methoxy group;
(3) the pharmaceutical composition according to (1), wherein the compound represented by the general formula (I) is a compound selected from the group consisting of
   (2R)-2-amino-2-methyl-4-{1-methyl-5-[4-(4-methylphenyl)butanoyl]pyrrol-2-yl}butan-1-ol,
   (2R)-2-amino-2-methyl-4-{1-methyl-5-[4-(3,4-dimethylphenyl)butanoyl]pyrrol-2-yl}butan-1-ol,
   (2R)-2-amino-2-methyl-4-{1-methyl-5-[4-(4-methoxy-3-methylphenyl)butanoyl]pyrrol-2-yl}butan-1-ol,
   (2R)-2-amino-2-methyl-4-[1-methyl-5-(5-phenylpentanoyl)pyrrol-2-yl]butan-1-ol, and
   (2R)-2-amino-2-methyl-4-[5-(5-phenylpentanoyl)thiophen-2-yl]butan-1-ol;
(4) the pharmaceutical composition according to any one of (1) to (3), wherein the hepatitis is T cell-dependent hepatitis, alcoholic hepatitis, or drug-induced hepatitis; and
(5) the pharmaceutical composition according to any one of (1) to (3), wherein the hepatitis is T cell-dependent hepatitis.

### Advantages of the Invention

The present invention can provide a pharmaceutical composition useful for prophylaxis or treatment of hepatitis. The pharmaceutical composition of the present invention is useful for prophylaxis or treatment of T cell-dependent hepatitis (viral hepatitis or autoimmune hepatitis), alcoholic hepatitis, or drug-induced hepatitis, preferably T cell-dependent hepatitis (viral hepatitis or autoimmune hepatitis). Moreover, the pharmaceutical composition is preferably intended for a warm-blooded animal, more preferably for a human.

### Best Mode for Carrying Out the Invention

The amino alcohol compound serving as an active ingredient of the present invention is a compound represented by the general formula (I): 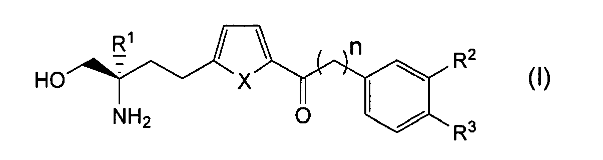 or a pharmacologically acceptable salt thereof.

In the formula,
R¹ represents a C₁-C₄ alkyl group,
R² and R³ which are the same or different represent a hydrogen atom, a C₁-C₄ alkyl group, or a C₁-C₄ alkoxy group,
X represents a sulfur atom or a nitrogen atom substituted by a methyl group, and
n represents an integer of 3 or 4.

The C₁-C₄ alkyl group of R¹, R², or R³ is, for example, a methyl, ethyl, propyl, isopropyl, butyl, or isobutyl group. The C₁-C₄ alkyl group of R¹, R², or R³ is preferably a methyl or ethyl group, particularly preferably a methyl group.

The C₁-C₄ alkoxy group of R² or R³ is, for example, a methoxy, ethoxy, propoxy, isopropoxy, butoxy, or isobutoxy group. The C₁-C₄ alkoxy group of R² or R³ is preferably a methoxy or ethoxy group, particularly preferably a methoxy group.

Moreover, the combination of the substituents is preferably the combination wherein R¹ is a methyl group, R² is a hydrogen atom, a methyl group, or a methoxy group, and R³ is a hydrogen atom, a methyl group, or a methoxy group. It is further preferred that R² and R³ should be different from each other.

Compounds represented by the general formula (I) are disclosed in, for example, International Publication No. WO 2005/079788 and can be produced by a method described in these documents.

The compound represented by the general formula (I) can be converted, if desired, to a corresponding pharmacologically acceptable salt by acid treatment according to a standard method. Such a "pharmacologically acceptable salt" is, for example: an inorganic acid salt such as halogenated hydroacid salts (e.g., hydrofluoride, hydrochloride, hydrobromide, and hydroiodide), nitrate, perchlorate, sulfate, and phosphate; an organic acid salt such as lower alkanesulfonic acid salts (e.g., methanesulfonate, trifluoromethanesulfonate, and ethanesulfonate), arylsulfonic acid salts (e.g., benzenesulfonate and p-toluenesulfonate), acetate, malate, fumarate, succinate, citrate, ascorbate, tartrate, oxalate, and maleate; and an amino acid salt such as glycine salt, lysine salt, arginine salt, ornithine salt, glutamate, and aspartate. The pharmacologically acceptable salt is preferably an inorganic or organic acid salt, more preferably hydrochloride, maleate, or fumarate.

The compound represented by the general formula (I) or the pharmacologically acceptable salt thereof, or the like, when left in atmosphere, may absorb moisture such that it has water adsorbed thereon or is converted to a hydrate. Such a compound or a salt is also used in the present invention.

Furthermore, the compound represented by the general formula (I) or the pharmacologically acceptable salt thereof, or the like, may absorb other certain solvents such that it is converted to a solvate. Such a compound or a salt is also used in the present invention.

The term "prophylaxis or treatment" in the present invention encompasses amelioration or cure of the disease as well as delay of disease progression, prophylaxis of onset, and prophylaxis of relapse. The term "prophylaxis or treatment" should not be considered restrictive by any means, and should be understood in the broadest sense.

It is preferred that the pharmaceutical composition of the present invention should be administered orally. Of course, the dosage form of the pharmaceutical composition of the present invention is not limited to oral administration, and it can also be administered parenterally, for example, through intravenous administration, intrarectal administration, transdermal administration, or transmucosal administration. Examples of unit dosage forms suitable for oral administration can include, but are not limited to, powders, granules, tablets, and capsules. For preparing the unit dosage forms, pharmacologically acceptable pharmaceutical additives can be used appropriately, such as excipients, lubricants, binders, disintegrants, emulsifiers, stabilizers, corrigents, and diluents.

Examples of the "excipients" used can include: organic excipients such as sugar derivatives (e.g., lactose, sucrose, glucose, mannitol, and sorbitol), starch derivatives (e.g., corn starch, potato starch, α-starch, and dextrin), cellulose derivatives (e.g., crystalline cellulose), gum arabic, dextran, and pullulan; and inorganic excipients such as silicate derivatives (e.g., light silicic anhydride, synthetic aluminum silicate, calcium silicate, and magnesium aluminometasilicate), phosphates (e.g., calcium hydrogen phosphate), carbonates (e.g., calcium carbonate), and sulfates (e.g., calcium sulfate).

Examples of the "lubricants" used can include: stearic acid; stearic acid metal salts such as calcium stearate and magnesium stearate; talc; colloidal silica; waxes such as beeswax and spermaceti; boric acid; adipic acid; sulfates such as sodium sulfate; glycol; fumaric acid; sodium benzoate; D,L-leucine; lauryl sulfates such as sodium lauryl sulfate and magnesium lauryl sulfate; silicic acids such as silicic anhydride and silicic hydrate; and the starch derivatives exemplified above.

Examples of the "binders" used can include hydroxypropylcellulose, hydroxypropylmethylcellulose, polyvinylpyrrolidone, macrogol, and the same compounds as those exemplified as the excipients.

Examples of the "disintegrants" used can include: cellulose derivatives such as low substituted hydroxypropylcellulose, carboxymethylcellulose, calcium carboxymethylcellulose, and internally cross-linked sodium carboxymethylcellulose; cross-linked polyvinylpyrrolidone; and chemically modified starches and celluloses such as carboxymethyl starch and sodium carboxymethyl starch.

Examples of the "emulsifiers" used can include: colloidal clay such as bentonite and veegum; metal hydroxides such as magnesium hydroxide and aluminum hydroxide; anionic surfactants such as sodium lauryl sulfate and calcium stearate; cationic surfactants such as benzalkonium chloride; and nonionic surfactants such as polyoxyethylene alkyl ether, polyoxyethylene sorbitan fatty acid ester, and sucrose fatty acid ester.

Examples of the "stabilizers" used can include: p-hydroxybenzoic acid esters such as methylparaben and propylparaben; alcohols such as chlorobutanol, benzyl alcohol, and phenyl ethyl alcohol; benzalkonium chloride; phenols such as phenol and cresol; thimerosal; dehydroacetic acid; and sorbic acid.

Examples of the "corrigents" used can include: sweeteners such as sodium saccharin and aspartame; acidulants such citric acid, malic acid, and tartaric acid; and flavors such as menthol, lemon, and orange.

Examples of the "diluents" used can include those usually used as diluents, such as lactose, mannitol, glucose, sucrose, calcium sulfate, calcium phosphate, hydroxypropylcellulose, microcrystalline cellulose, water, ethanol, polyethylene glycol, propylene glycol, glycerol, starch, polyvinylpyrrolidone, magnesium aluminometasilicate, and mixtures thereof.

The dose of the pharmaceutical composition of the present invention can be selected appropriately according to various factors such as the administration route, the type of the active ingredient, the age, body weight, or symptoms of a patient, and a prophylactic or therapeutic purpose, and is generally 0.0005 mg/kg (preferably 0.001 mg/kg) as the lower limit, and 0.1 mg/kg (preferably 0.02 mg/kg) as the upper limit, per day for a human adult (body weight: approximately 60 kg).

The frequency of administration of the pharmaceutical composition of the present invention is usually once daily. Depending on symptoms, it can also be administered by administering a few days' doses at a time every few days or by administering a daily dose in divided doses several times daily.

### Examples

Hereinafter, the present invention will be described more specifically with reference to Examples and Preparation Examples. However, the scope of the present invention is not limited to these.

### (Test Example)

### (1) Objective

Aspartate aminotransferase (AST) and alanine aminotransferase (ALT) in serum were measured as indices for liver injury in concanavalin A (ConA)-induced mouse hepatitis models used as T cell-dependent liver injury models. The inhibitory effects of compounds of the present invention on liver injury were studied.

### (2) Method

A compound suspension was orally administered at a dose of 1 mg/kg to male BALB/c mice. 24 hours after the oral administration, ConA was intravenously administered at a dose of 20 mg/kg to the tails of the mice. 24 hours after the ConA administration, blood was collected from the mice, and AST and ALT in serum were measured.

### (3) Results

The administration of all the administered compounds (compounds 1 to 5) exhibited significant inhibitory effects on AST level elevating effects. Moreover, the administration of the compounds 1, 2, 4, and 5 was confirmed to have significant inhibitory effects on ALT level elevating effects, and the administration of the compound 3 was also confirmed to have a tendency to inhibit ALT level elevating effects. The administration of the compounds of the present invention was demonstrated to reduce both the AST and ALT levels serving as indices for liver injury. All things considered, the compounds of the present invention could be determined to inhibit liver injury.

**(Table 1)**

| Compound No. | AST level (mean ± S.E., IU/L) |
|---|---|
| Negative control | 105.2 ± 26.1 |
| Solvent | 236.9 ± 52.6 |
| Compound 1 | 97.4 ± 9.1** |
| Compound 2 | 115.4 ± 14.6* |
| Compound 3 | 123.6 ± 27.9* |
| Compound 4 | 95.2 ± 11.3** |
| Compound 5 | 109.1 ± 21.0** |

| | |
|---|---|
| (**P<0.01, *P<0.05; vs. solvent) | |

**(Table 2)**

| Compound No. | ALT level (mean ± S.E., IU/L) |
|---|---|
| Negative control | 76.4 ± 14.2 |
| Solvent | 454.1 ± 137.8 |
| Compound 1 | 101.6 ± 19.6** |
| Compound 2 | 182.5 ± 33.8* |
| Compound 3 | 227.0 ± 91.4 |
| Compound 4 | 108.7 ± 22.2** |
| Compound 5 | 127.5 ± 27.4** |

| | |
|---|---|
| (**P<0.01, *P<0.05; vs. solvent) | |

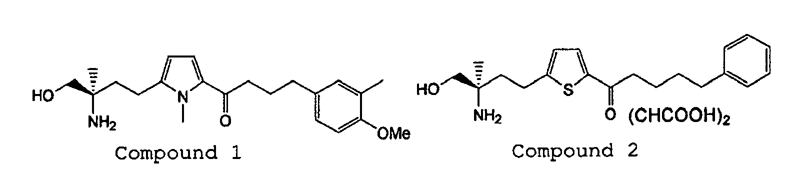

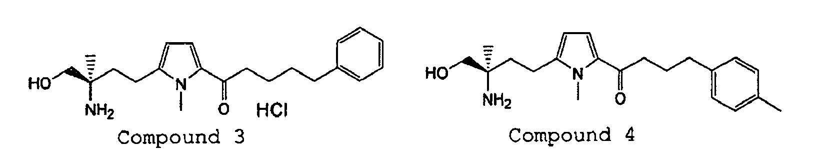

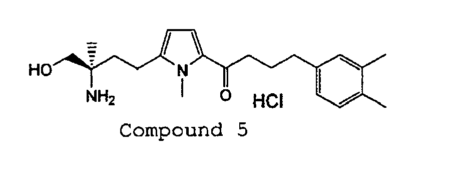

### (4) Discussion

Based on these results, the pharmaceutical composition of the present invention inhibits liver injury and is therefore likely to be useful for prophylaxis or treatment of T cell-dependent hepatitis (viral hepatitis or autoimmune hepatitis), alcoholic hepatitis, or drug-induced hepatitis, preferably T cell-dependent hepatitis (viral hepatitis or autoimmune hepatitis). Moreover, in consideration of its mechanism of action, the combined use of the pharmaceutical composition of the present invention with an additional preparation whose main target is not T cells is likely to increase further its prophylactic or therapeutic effects.

| (Preparation Example 1) Capsule | |
|---|---|
| Compound as active ingredient | 1.0 mg |
| Lactose | 158.7 mg |
| Corn starch | 70.0 mg |
| Magnesium stearate | 1.3 mg |
| Total | 250.0 mg |

A powder thus formulated is mixed and passed through a 60-mesh sieve. Then, this powder is placed into a gelatin capsule shell to prepare a capsule.

| (Preparation Example 2) Tablet | |
|---|---|
| Compound as active ingredient | 1.0 mg |
| Lactose | 154.0 mg |
| Corn starch | 25.0 mg |
| Magnesium stearate | 1.0 mg |
| Total | 200.0 mg |

A powder thus formulated is mixed and tableted using a tableting machine to prepare a tablet (200 mg per tablet). This tablet can be sugar-coated, if necessary. Industrial Applicability

The present invention can provide a pharmaceutical composition useful for prophylaxis or treatment of hepatitis. The pharmaceutical composition of the present invention is useful for prophylaxis or treatment of T cell-dependent hepatitis (viral hepatitis or autoimmune hepatitis), alcoholic hepatitis, or drug-induced hepatitis, preferably T cell-dependent hepatitis (viral hepatitis or autoimmune hepatitis). Moreover, the pharmaceutical composition is preferably intended for a warm-blooded animal, more preferably for a human.

## Claims

1. A pharmaceutical composition for prophylaxis or treatment of hepatitis, comprising a compound represented by the general formula (I): 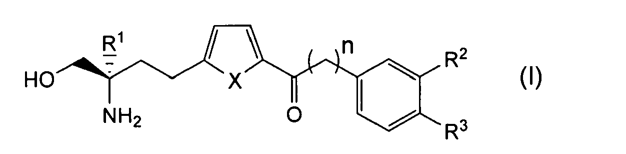 wherein
R¹ represents a C₁-C₄ alkyl group,
R² and R³ which are the same or different represent a hydrogen atom, a C₁-C₄ alkyl group, or a C₁-C₄ alkoxy group,
X represents a sulfur atom or a nitrogen atom substituted by a methyl group, and
n represents an integer of 3 or 4,
or a pharmacologically acceptable salt thereof, as an active ingredient.

2. The pharmaceutical composition according to claim 1, wherein R¹ is a methyl group, and R² and R³ which are the same or different are a hydrogen atom, a methyl group, or a methoxy group.

3. The pharmaceutical composition according to claim 1, wherein the compound represented by the general formula (I) is a compound selected from the group consisting of
(2R)-2-amino-2-methyl-4-{1-methyl-5-[4-(4-methylphenyl)butanoyl]pyrrol-2-yl}butan-1-ol,
(2R)-2-amino-2-methyl-4-{1-methyl-5-[4-(3,4-dimethylphenyl)butanoyl]pyrrol-2-yl}butan-1-ol,
(2R)-2-amino-2-methyl-4-{1-methyl-5-[4-(4-methoxy-3-methylphenyl)butanoyl]pyrrol-2-yl}butan-1-ol,
(2R)-2-amino-2-methyl-4-[1-methyl-5-(5-phenylpentanoyl)pyrrol-2-yl]butan-1-ol, and
(2R)-2-amino-2-methyl-4-[5-(5-phenylpentanoyl)thiophen-2-yl]butan-1-ol.

4. The pharmaceutical composition according to any one of claims 1 to 3, wherein the hepatitis is T cell-dependent hepatitis, alcoholic hepatitis, or drug-induced hepatitis.

5. The pharmaceutical composition according to any one of claims 1 to 3, wherein the hepatitis is T cell-dependent hepatitis.
